# EUROPEAN PATENT SPECIFICATION

(11) **EP 3 694 962 B1**
(45) Date of publication and mention of the grant of the patent: **13.12.2023**
(21) Application number: 18865493.3
(22) Date of filing: 12.10.2018
(51) Int. Cl.: C11C 3/02, C12P 7/64, C12N 9/20, A23L 33/115, C11C 1/04

(54) **PROCESSED OIL COMPRISING MONOACYLGLYCERIDES**
VERARBEITETES ÖL MIT MONOACYLGLYCERIDEN
HUILE TRANSFORMÉE COMPRENANT DES MONOACYLGLYCÉRIDES

(30) Priority: 13.10.2017 US 201762571910 P
(43) Date of publication of application: 19.08.2020
(60) Divisional application: 23207425.2
(73) Proprietor: GlycosBio Inc., Houston, TX 77021 (US)
(72) Inventor: MONTICELLO, Daniel J., The Woodlands TX 77381 (US); BUSSMANN, Werner J., Houston TX 77062 (US)
(74) Representative: Gill Jennings & Every LLP
(86) International application number: PCT/US2018/055583
(87) International publication number: WO 2019/075307

(56) References cited:
- WO-A1-2015/069974
- WO-A1-2016/066460
- WO-A1-2016/066460
- WO-A1-2017/019872
- US-A1- 2003 054 082
- US-A1- 2004 062 847
- ROSU et al.: "Repeated Use of Immobilized Lipase for Monoacylglycerol Production by Solid- Phase Glycerolysis of Olive Oil", JAOCS, vol. 74, no. 4, 1997, pages 445-450, XP055592138,
- APARICIO et al.: "Characterization of Monovarietal Virgin Olive Oils", Eur. J. Lipid Sci. Technol., vol. 104, no. 9-10, 2002, pages 614-627, XP055592144, ISSN: 1438-7697
- JIANG et al.: "Magnetic Nanoparticles supported ionic liquids for lipase mobilization: Enzyme activity in catalyzing esterification", Joumal of Molecular Catalysis B: Enzymatic, vol. 58, 2009, pages 103-109, XP026062997, DOI: doi:10.1016/j.molcatb.2008.12.001
- CHUN ET AL: "Tocopherol and tocotrienol contents of raw and processed fruits and vegetables in the United States diet", JOURNAL OF FOOD COMPOSITION AND ANALYSIS, ELSEVIER, AMSTERDAM, NL, vol. 19, no. 2-3, 1 March 2006 (2006-03-01), pages 196-204, XP005197534, ISSN: 0889-1575, DOI: 10.1016/J.JFCA.2005.08.001

## Description

### BACKGROUND

Chronic deficiency in the secretion of digestive enzymes by the pancreas is termed Exocrine Pancreatic Insufficiency (EPI). Without these digestive enzymes, patients suffering from EPI cannot properly digest nutrients in food and may suffer from malnutrition and abdominal disorders. EPI is prevalent in individuals with chronic pancreatitis and several other chronic gastrointestinal disorders. EPI also manifests in patients suffering from cystic fibrosis. The effects of EPI can be mitigated by Pancreatic Enzyme Replacement Therapy (PERT), in which the individual administers enzyme capsules each time food is consumed. Conventionally, PERT treatment comprises pancreatic enzymes extracted from porcine pancreas.

Lipids are energy-dense compounds that are the source of essential long chain fatty acids. Consumed lipids are digested with lipases secreted from the pancreas into free fatty acids (FFA) and monoacylglyceride (MAG). Blockage of lipase release from the pancreas results in very poor digestion of fats and oils. For patients suffering from EPI, this can lead to significant malnutrition because the calories, essential fatty acids and fat-soluble nutrients are trapped in the un-digested lipid particles and pass through the system.

There is an un-met clinical need for alternative sources of nutrition that can be consumed without needing to supplement with PERT by individuals with EPI.

Partially hydrolyzed fats and oils, in the form of MAGs are readily absorbed by individuals with EPI, without the requirement of PERT. MAG oil-based products have been evaluated in the clinic as capsule-based nutritional supplements; however, capsules were utilized to avoid the bad taste. For conventional sources of MAG oils, the starting oil is treated chemically or enzymatically to make MAGs, which are then extracted with solvents and distilled to fractionate the MAGs away from other components of the starting oils. These MAG products are sold as relatively pure products containing only small amounts of contaminating FFA, DAGs and TAGs, and with virtually no other compounds. Thus, conventional sources of MAG oil often lack the other natural compounds found in the oil, such as tocopherol.

There is clinical need for nutritional products with very-high-energy caloric density that can be consumed by individuals with inefficient or compromised digestive systems. In addition to individuals with pancreas pathologies (e.g., cystic fibrosis, pancreatitis and pancreatic cancer patients) other patients with diagnosed or undiagnosed Exocrine Pancreatic insufficiency (EPI) would benefit from the products. In addition, individuals with bile dysfunction (cholestasis) may benefit from "pre-digested" fats that do not require bile acids for emulsification. There are high calorie "energy bars" and drinks on the market. But, these products are not suitable for individuals who are unable to digest (hydrolyze) the fats in the product. Until now, no one has formulated lipids into liquid (shakes) and solid (bar) forms that are suitable for "PERT-free" use.

Accordingly, there is need for high caloric density foods that can be consumed by individuals with inefficient or compromised digestive systems. The present application describes a method to produce an edible enzyme-modified oil (EMO) that is substantially free of triacylglycerides (TAGs).

WO 2017/019872 A1 describes a MDG oil comprising 40-60% monoglycerides. The MDG oil may comprise triglycerides.

WO 2015/069974 A1 describes a nutritional supplement formulation, comprising a lipophilic nutrient, and the lipophilic MDG carrier oil.

US 2004/06284 7 A1 describes an oil/fat composition comprising 5 to 99.9 wt.% of a monoglyceride.

US 2003/054082 A1 discloses an oil or fat composition containing a monoacylglycerol and/or a diacylglycerol in a total amount of 5-100 wt. % and which exhibits an index of stability against oxidation (induction time as measured through a Rancimat test conducted at 100° C.) of 7 or higher, wherein the monoacylglycerol and/or the diacylglycerol contain, as fatty acid constituents, ω3 unsaturated fatty acids in amounts of 15-90 wt. %.

WO 2016/066460 A1 describes a nutritional composition comprising sn-I(3) monoacylglycerols for use in the treatment or prevention of maldigestion and/or malabsorption in an infant or young child.

### SUMMARY

The present disclosure is directed to a processed oil product derived from an oil source, wherein the processed oil comprises (1) a MAG content equal to or greater than 40% by weight of the total weight of the processed oil; (2) a TAG content equal to or lesser than 5% by weight of the total weight of the processed oil, and (3) non-oil ingredients derived from and naturally present in the oil source, such that said non-oil ingredients are not added to the processed oil, wherein the non-oil ingredients are selected from α-tocopherol, β-tocopherol, δ-tocopherol, γ-tocopherol, α-tocotrienol, β-tocotrienol, δ-tocotrienol, and γ-tocotrienol.

In some embodiments, the oil source of said product is from an origin selected from a plant, an animal, or a fish.

In some embodiments the non-oil ingredients of said product are selected from antioxidants, vitamins, and mixtures thereof.

In some embodiments, said product comprises greater than 1% by weight MAGs out of the total weight of the product.

In some embodiments, said product comprises greater than 50% by weight MAGs out of the total weight of the product.

In some embodiments, the oil source is from an origin selected from a plant, an animal, or a fish.

In some embodiments, the non-oil ingredients are selected from antioxidants, vitamins, and mixtures thereof.

### BRIEF DESCRIPTION OF THE DRAWINGS AND FIGURES

FIGURE 1 depicts the TLC separation of components of starting vegetable oil, intermediate FFAs, and final MAG oil.
FIGURE 2 depicts the TLC separation of components of starting vegetable oil, intermediate FFAs, and final MAG oil.
FIGURE 3 depicts the distribution of FFA, MAG, DAG, and TAG in "Ensure Original Nutritional Shake" and Enzyme Modified Oil product of the present disclosure ("GBFS").
FIGURE 4 depicts a block flow diagram of the process of manufacturing enzyme modified oil.
FIGURE 5 depicts the distribution of amino acids and peptides in ready-to-drink nutritional drinks and GBFS hydrolyzed pea protein.
FIGURE 6A depicts the NMR spectrum of authentic TAG (tristerin).
FIGURE 6B depicts the NMR spectrum for enzyme modified oil produced from almond oil.

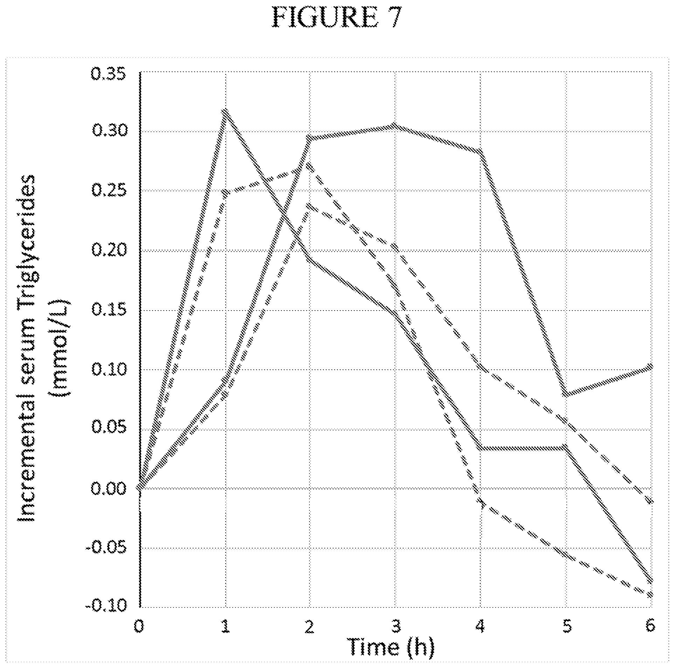

FIGURE 7 depicts the increase in serum triglycerides following ingestion of the EMO-based ready-to-drink shake.

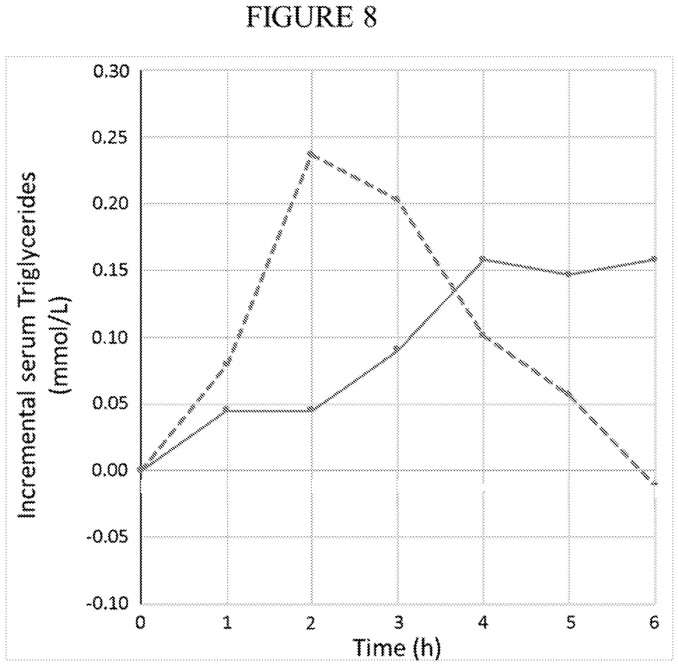

FIGURE 8 depicts the increase in serum triglycerides following ingestion of MAG-based RTDS without PERT.

### DETAILED DESCRIPTION OF THE DISCLOSED SUBJECT MATTER

It is to be understood that both the foregoing general description and the following detailed description are exemplary and explanatory only, and are not restrictive of the invention, as claimed. In this application, the use of the singular includes the plural, the word "a" or "an" means "at least one", and the use of "or" means "and/or", unless specifically stated otherwise. Furthermore, the use of the term "including", as well as other forms, such as "includes" and "included", is not limiting. Also, terms such as "element" or "component" encompass both elements or components comprising one unit and elements or components that comprise more than one unit unless specifically stated otherwise.

The section headings used herein are for organizational purposes only and are not to be construed as limiting the subject matter described. All documents, or portions of documents, cited in this application, including, but not limited to, patents, patent applications, articles, books, and treatises, are hereby expressly incorporated herein by reference in their entirety for any purpose. In the event that one or more of the incorporated literature and similar materials defines a term in a manner that contradicts the definition of that term in this application, this application controls.

"Enriched" in the context of this invention means with an amount higher than in the starting material. For example, a MAG-enriched oil is an oil having a MAG content that is greater than the starting MAG content prior to the enrichment process or that the starting oil has a greater percentage of MAG than the oil possessed prior to the enrichment process. The enrichment process can be by conversion of TAGs to MAGs thereby increasing the MAG content and percentage and decreasing the TAG content or percentage.

A triacylglycerol ("TAG"), also known as a triglyceride, is a glyceride consisting of three fatty acid chains covalently bonded to a glycerol molecule through ester linkages. TAGs may also be classified as having a long or medium chain length. Long chain TAGs contain fatty acids with 14 or more carbons, while medium chain TAGs contain fatty acids with 6 to 12 carbons. Long chain TAGs can include omega-3 and omega-6 fatty acids. Medium chain TAGs have saturated fatty acids and thus do not contain omega-6 or omega-3 fatty acids. Long chain TAGs (LCT) and medium chain triglycerides (MCT) can serve as energy sources.

A diacylglycerol ("DAG"), also known as a diglyceride, is a glyceride consisting of two fatty acid chains covalently bonded to a glycerol molecule through ester linkages.

A monoacylglycerol ("MAG"), also known as a monoglyceride, is a glyceride consisting of one fatty acid chain covalently bonded to a glycerol molecule through an ester linkage
As used herein, the term "processed oil" refers to a non-naturally occurring oil composition substantially free of triacylglycerols ("TAGs") or having a reduced amount of TAGs with respect to the pre-modified or pre-processed oil.

As used herein, a "non-oil ingredient" is an ingredient that is naturally present in an oil source that is not a MAG, DAG, TAG, FFA or lipid.

In some embodiments, the starting oils may comprise, by way of example but not limitation, oils derived from plants such as olive oil, almond oil flaxseed oil, sunflower seed oil, corn oil, rapeseed oil, palm oil, soy bean oil, or oil derived from animals such as fish oil, sardine oil, or anchovy oil, or algal oil, or mixtures thereof. In one aspect, the starting oil comprises a blend of olive oil, sunflower seed oil, and flaxseed oil, wherein the from about 50% to about 80% by weight of the total weight of the starting oil is olive oil, from about 10% to about 30% by weight of the total weight of the starting oil is suflower seed oil, and from about 5% to about 20% by weight of the total weight of the starting oil is flaxseed oil. In another aspect, from about 50% to about 80% by weight of the total weight of the starting oil is olive oil, from about 10% to about 30% by weight of the total weight of the starting oil is flax seed oil, and from about 5% to about 20% by weight of the total weight of the starting oil is sunflower seed oil.

The processed oil has a MAG content of equal to or greater than 40% by weight based on the total weight of the processed oil. In certain aspects, the MAG content is from about 40% to about 99% by weight based on the total weight of the processed oil. In certain aspects, the MAG content is from about 50% to about 99% by weight based on the total weight of the processed oil. In certain aspects, the MAG content is from about 60% to about 99% by weight based on the total weight of the processed oil. In certain aspects, the MAG content is from about 70% to about 99% by weight based on the total weight of the processed oil. In certain aspects, the MAG content is from about 80% to about 99% by weight based on the total weight of the processed oil. In certain aspects, the MAG content is from about 50% to about 80% by weight based on the total weight of the processed oil. In any of the above aspects, the TAG content is equal to or less than 5% by weight based on the total weight of the processed oil. In any of the above aspects, the TAG content is equal to or less than 4%, equal to or less than 3%, equal to or less than 2%, equal to or less than 1% by weight based on the total weight of the processed oil.

The processed oil comprises a MAG content equal to or greater than 40% by weight of the total weight of the processed oil. By way of example but not limitation, the processed oil comprises a MAG content of about 40% to 95%, 50% to 95%, 60% to 95%, 70% to 95%, 80% to 95%, 90% to 95%, 40% to 90%, 50% to 90%, 60% to 90%, 70% to 90%, 80% to 90%, 40% to 80%, 50% to 80%, 60% to 80%, 70% to 80%, 40% to 70%, 50% to 70%, 60% to 70%, 40% to 60%, 50% to 60%, 40% to 50%, or about 40%, 45%, 50%, 55%, 60%, 65%, 70%, 75%, 80%, 85%, 90%, or 95% by weight of the total weight of the processed oil.

The processed oil is either free of TAGs or comprises a TAG content that is equal to or less than 5% by weight of the total weight of the processed oil. By way of example but not limitation, the processed oil comprises a TAG content that is about 0% to 5%, 1% to 5%, 2% to 5%, 3% to 5%, 4% to 5%, 0% to 4%, 1% to 4%, 2% to 4%, 3% to 4%, 0% to 3%, 1% to 3%, 2% to 3%, 0% to 2%, 1% to 2%, 0% to 1%, or 0%, 1%, 2%, 3%, 4% or 5% by weight of the total weight of the processed oil. By way of further example but not limitation, the TAG content can be less than 5%, 4%, 3%, 2%, 1%, 0.5%, or 0.1%.

The processed oil comprises non-oil ingredients derived from and naturally present in the oil source such that the non-oil ingredients are not added to the processed oil, wherein the non-oil ingredients are selected from alpha-tocopherol, beta-tocopherol, delta-tocopherol, gamma-tocopherol, alpha-tocotrienol, beta-tocotrienol, delta-tocotrienol, or gamma-tocotrienol.

### EXAMPLES

### Example 1: Process of making a product enriched in MAG compared to starting TAG-rich oil.

The method of making a product enriched in MAG compared to the starting TAG-rich oil involves 3 key steps: (1) A mild enzymatically-catalyzed reaction to hydrolyze triglycerides (TAGs) in a sequence that converts the neutral oils to specific combinations of FFAs, MAGs, DAGs, and low residual TAGs; (2) an esterification with glycerol to generate predominantly high amounts of MAGs leaving low concentrations of FFAs; and (3) isolation of the modified lipid product; this is achieved by phase separation with or without the aid of a centrifuge.

### Step 1. Enzymatic Conversion of Triacylglycerols

**Preparation of the buffer solution.** A sodium citrate solution (100 mM, pH 5.8) was prepared in a stirred tank reactor. 11.1 L of deionized (DI) water was placed in the mixing vessel, the agitator was set to 200 RPM and 0.213 kg of citric acid (anhydrous) were added. After the powder was dissolved the pH was adjusted with sodium hydroxide solution to pH = 5.8 (about 0.121 kg). 220 mL was removed for subsequent enzyme preparation.

**Preparation of the enzyme solution**. In a separate 250 mL bottle, the enzyme solution was prepared under gentle stirring: 200 mL of the citrate buffer solution was placed in the mixing vessel. 10 g of AMANO Lipase AY was added and the bottle was shaken until the enzyme is dissolved.

**Conversion of Triglyceride Oil Mixture**. Three vegetable oils, Olive Oil, Flaxseed Oil and Sunflower seed Oil were added to the vessel to give a total of 10 kg of plant oil mixture. Vacuum was applied to drop the pressure to about 20 mmHg and degas the material (from any dissolved oxygen in particular). Agitation was set to 200 RPM and the mixture was heated to 33 °C and agitated for about 15 Min to remove any dissolved gas. The vacuum was then replaced by a Nitrogen gas atmosphere. Once the mixture was sufficiently dispersed, 220 mL of the enzyme preparation in buffer solution was added. Agitation was continued and the reaction monitored for 24 hours until conversion to FFA was complete based on TLC analysis.

Reactor temperature was increased to 70° C and agitation resumed for 1 hour to inactivate the enzyme.

Agitation was stopped and the phases separated in about 60 Minutes.

The aqueous (lower) phase was removed along with a small amount of the oil phase to ensure residual protein at the interface was eliminated.

**Step 2. Esterification with Glycerol.** Typically when FFA are re-esterified with glycerol they produce a mixture of MAGs, DAGs and TAGs. We found that by significantly reducing the temperature (below 25°C) and removing the water (by evaporation) as it is formed in the reaction, that the proportion of MAGs in the product can be highly enriched (at least 60% but as high as 95%). This was un-expected.

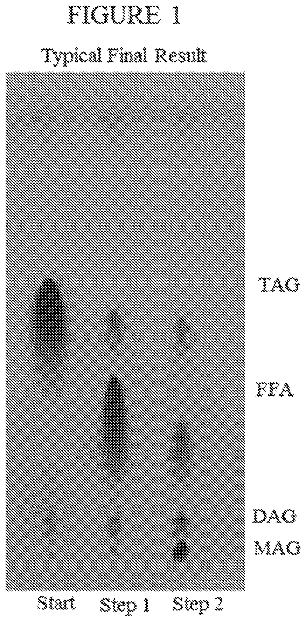

In the reaction, the reaction product from step 1 (about 10L) was cooled to about 30° C and agitated at 300 RPM. 10 kg of Food-grade glycerol were added to the Lipid mixture, and the temperature maintained at ~ 30° C. The mixture was agitated to generate a dispersion of the oil and glycerol. To dry the reaction mixture a vacuum was applied: First a vacuum (25 mmHg, Torr) was applied with receiver in place to collect water. Once evaporation of the residual water had stopped, 20g of Amano Lipase G dissolved in water (50 mL) was added to the reactor. The temperature was lowered to 23°C and the vacuum was changed to 5 mmHg using an oil diaphragm pump and a cold trap to collect the water. The mixture was stirred at 300 RPM at 23°C under vacuum for 72 hrs, at which time the vacuum was broken and the mixture was blanketed with nitrogen gas. The mixture was analyzed after 72 hrs with TLC to evaluate the conversion to MAGs as shown in FIGURE 1.

**Step 3. Lipase Inactivation and Phase Separation.** After the reaction was complete, the lipase was inactivated by heating the mixture (under a Nitrogen gas blanket) to 70°C for 1 hr. At this point the MAG oil and glycerol are well mixed and very hard to separate through traditional gravity or centrifugal methods. After considerable experimentation, we found that the lipid can be separated from the excess glycerol by adding 0.3%wt of salt (NaCl) to the reaction mixture under agitation. The product mixture was then allowed to cool to about 60°C and left without agitation for about 1 hrs.

The lipid oil phase separated from the remaining heavier glycerol phase. The glycerol phase was removed: it contains some salt, residual water and the dissolved inactivated enzyme which is contained in the visible interface. The glycerol phase can be reused after membrane filtration and should be kept for recycle. Tocopherol (Vitamin E) was added to give a concentration of 200 ppm (0.02% wt) of the product oil. The hydrolyzed oil (about 10 kg was ready for use and could be stored under nitrogen blanket).

**Product Storage**. The final hydrolyzed products was transferred to food-grade containers with an overlay of nitrogen gas for storage and transportation.

### Example 2: Characterization of oil produced in Example 1.

Reaction products and the overall process can be evaluated using Thin Layer Chromatography and Gas Chromatography.

**Thin-Layer Chromatography Testing**. Components of the oil samples were separated using TLC plates (Analtech Uniplate Silica Gel GHL with inorganic binder, 20 x 20 cm, 250 µm). The solvent was Hexane: Diethyl Ether: Acetic Acid (70:30:1) solution. Typical sample sizes were 3 µL. After the solvent front ran to near the top of the plate (~1 cm), plates were removed from the TLC tank and the solvent evaporated in a fume hood. The components were visualized with iodine vapors (at room temperature) in a TLC tank and relative intensities estimated by colorimetric imaging (Amersham 600 Imager). After 15 minutes in the tank, plates were removed and photographed. The intensity of the spots diminished after 30 - 60 minutes.

Physical properties determination was performed to establish product consistency, color, water content, fats & oils (miscible).

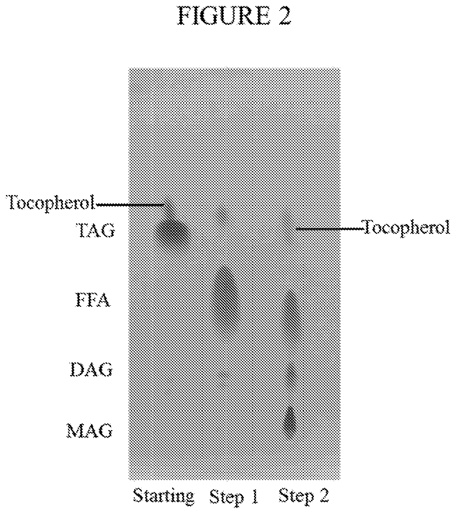

FIGURE 1 depicts the final results of steps 1 to 3 in the process of making a product enriched in MAG compared to starting TAG-rich oil as describe in Example 1. FIGURE 2 illustrates that tocopherol initially present in the olive oil is preserved following the steps described in Example 1. In FIGURE 2, the tocopherol spot can be seen tracking above the TAG spot in all three lanes.

**Fatty Acid Profile Testing - Gas Chromatography**. Lipid components including C10:0 Capric Acid, C12:0 Lauric Acid, C14:0 Myristic Acid, C16:0 Palmitic Acid, C18:0 Stearic Acid, C18:1 Oleic Acid, C18:2 Linoleic Acid, and C18:3 Alpha Linolenic Acid were analyzed after derivatization as the fatty acid methyl esters and compared to standards. For the derivatization, a sample (500 µl) was added to a 5-ml reaction tube containing 2 ml boron trifluoride solution (12% in methanol), 20 µl dimethoxypropane and 100 µl of a tridecanoic acid internal standard solution (10 mg/ml). The reaction tube was vortexed and incubated in a heating block at 60°C for 30 minutes.

The reaction tube was removed from the heating block and allowed to cool for 15 minutes. Then, 1 ml of distilled water was added to quench the reaction, followed by 1 ml of hexane. The reaction tube was vortexed for 60 seconds and the phases were allowed to separate for 3 minutes. The top (hydrophobic) phase was removed to a 1.5-ml tube containing about 50 mg sodium sulfate (anhydrous). After vortexing for 60 seconds, the 1.5-ml tube was centrifuged and ~500 µl of the clarified, dried hydrophobic phase was transferred to a gas chromatography sample vial.

Samples were analyzed using an Agilent 7890A gas chromatograph with Flame ionization detector and Agilent Openlab CDS Chemstation software. GC Column: Omegawax 100 (15m x 0.1 mm x 0.1 um) column. Results were converted to weight % by internal standard reference.

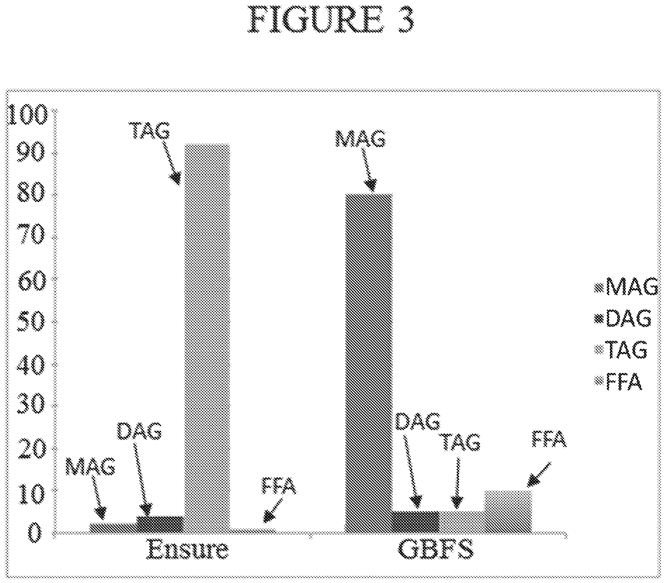

FIGURE 3 depicts the distribution of FFA, MAG, DAG, and TAG in Ensure and a GBFS product of the present disclosure. Percent FFA, MAG, DAG and TAG in the oils determined by Thin Layer Chromatography.

### Example 3: Another example of the process of making a product enriched in MAG compared to starting TAG-rich oil.

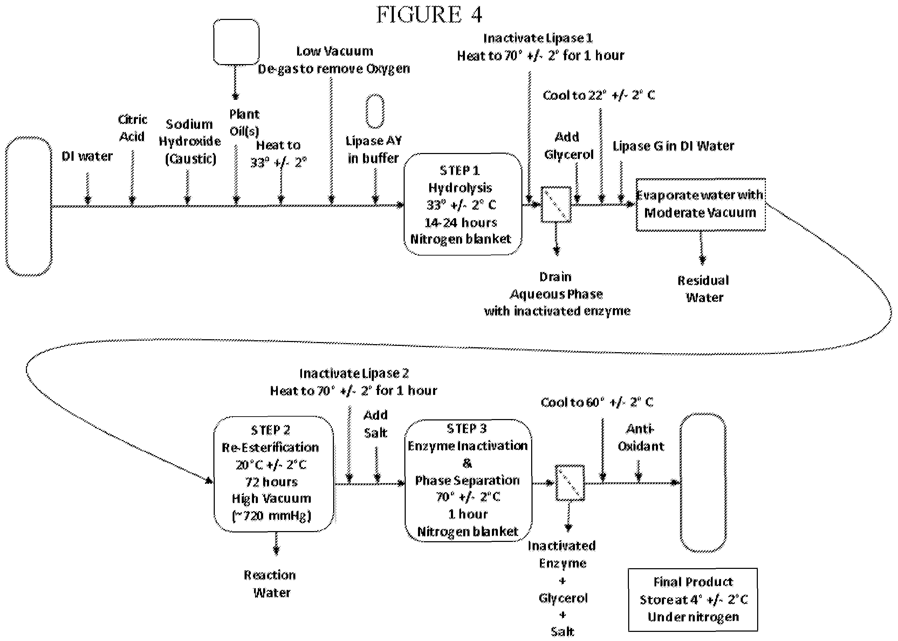

The following procedure is depicted by block flow diagram in FIGURE 4.

Plant oil was added to citric acid and sodium hydroxide (caustic) in DI water and heated to 33 °C +/- 2 °C. A low vacuum was applied to de-gas to remove oxygen. Lipase AY was added. Hydrolysis of the mixture was performed for 14 - 24 hours under a Nitrogen blanket at a temperature of 33 °C +/- 2 °C.

Lipase was inactivated at a temperature of 70 °C +/- 2 °C for 1 hour. The aqueous phase with inactivated enzyme was drained. Glycerol was added. Then the reaction was cooled to 22 °C +/- 2 °C. Then Lipase G was added and water was evaporated under a moderate vacuum.

Re-esterification was carried out for 72 hours under high vacuum (around 720 mmHg) at a temperature of 20 °C +/- 2 °C. Lipase G was inactivated at 70 °C +/- 2 °C for one hour and salt was added to the reaction.

Enzyme inactivation and phase separation was carried out under a Nitrogen blanket for 1 hour at 70 °C +/- 2 °C. The aqueous phase with inactivated enzyme, glycerol, and salt was drained. The reaction was cooled to 60 °C +/- 2 °C. Antioxidant was added. The final product was stored at 4 °C +/- 2 °C under nitrogen.

### Example 4: Ready-to-Drink formulation incorporating MAGs and hydrolyzed protein

A product of the present disclosure was produced as a conventional "milk shake" formulation that includes a source of fats, proteins, carbohydrates, vitamins and fiber in addition to the traditional surfactants and stabilizing agents typically found in these products. An individual serving was 250 ml. The ingredients as would appear on the ingredient label were as follows: Water, Organic Agave Syrup, Hydrolyzed Pea Protein, Hydrolyzed Oil Blend, Gum Arabic, Sunflower Lecithin, Xanthan Gum, Oligosaccharides, Potassium Sorbate, Sodium Benzoate, Instant Coffee, Natural Organic Vanilla Flavor, Vitamin C, Vitamin E Succinate, Vitamin A Palmitate, Niacinamide, D-Calcium Pantothinate, Pyrodoxine HCl, Thiamine HCl, Riboflavin, Vitamin D3, Folic Acid, Cyanocobalamin, Vitamin K2.

Carbohydrates were supplied as simple sugars (glucose and fructose) from fruit and agave syrups.

Protein in the product was partially hydrolyzed pea protein (PURIS Pea Protein 870H, World Food Processing LLC, Turtle Lake, WI 54889).

We also produced extensively hydrolyzed pea protein (EHP) with peptides in a size range more bio-available for transport across the intestinal wall. EHP was produced by further enzyme hydrolysis. For example, partially hydrolyzed pea protein (Puris Pea Protein 870H described above) protein was dissolved in 100 mM Phosphate buffer to a concentration of 25 mg/ml. Enzyme was added and the reaction incubated at 50° overnight. Three different commercial GRAS enzymes were evaluated: Alcalase, Thermoase and flavourzyme.

The average size and distribution of peptides in the protein samples were evaluated using size exclusion chromatography. Samples were dissolved in 100 mM phosphate buffer, pH 6.8 to a concentration of 25 mg/ml and analyzed on a Shimadzu HPLC with UV detector (214 nm) using a Phenomenex Yarra 3 um SEC-2000 column (Eluted with 100 mM sodium Phosphate buffer (pH 6.8), Flow rate of 0.8 mL/min at room temperature). Samples were compared to a molecular weight standard (Phenomenex SEC standard part ALO-3042). Sizes were estimated using a calibration curve generated from known molecular weight standards. The average size of un-hydrolyzed pea protein was ~200 amino acids. The average size of the Puris Pea 870H partially hydrolyzed material was 34 amino acids, with a substantial amount in the 2-40 amino acid range, similar to other (whey-based) protein hydrolysate products.

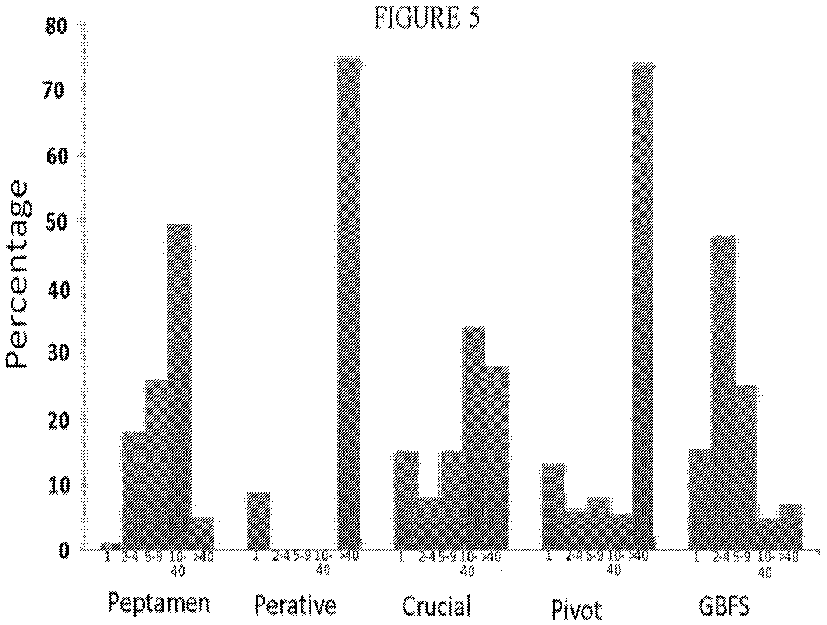

FIGURE 5 depicts the distribution of amino acids and peptides in ready-to-drink nutritional drinks as reported in E. Phillips et al., 2005 Peptide-Based Formulas: the Nutraceuticals of Enteral Feeding? EPCN October:40-45 compared to the GBFS extensively hydrolyzed pea protein described above. FIGURE 5 depicts the percentage of amino acids (Y-Axis) of the size 1 amino acid, 2-4 amino acids, 9-10 amino acids, 10-40 amino acids, or greater than 40 amino acids in the products Oeotamen, Perative, Cricual, Pivot, and the GBFS of the present disclosure (X-Axis). The average size of the GBFS EHP is 3-4 amino acids, mostly 1-7 amino acids.

Fat is provided in the form of the re-structured Lipid MAG and were produced from a blend of olive oil (70%), sunflower oil (21%) and flax seed oil (9%) to provide the energy and other benefits of poly-unsaturated fatty acids (PUFA), omega-6 PUFA and omega-3 PUFA. The Omega-6/omega-3 ratio is ~4/1.

Gum Arabic, sunflower lecithin, and xanthan gum are common GRAS food ingredients used to provides structure and stability for the drink. Oligosaccharides provide non-digestable fiber. Potassium Sorbate and Sodium Benzoate are common food preservatives. Instant coffee and Vanilla provide flavor.

A vitamin package including fat soluble and water-soluble vitamins was included.

A prototype Product Nutritional label is shown in Table 1. In Table 1, ** Percentage Daily Values (% DV) are based on a 2,000 calorie diet. † Daily Value (DV) not established.

| **Supplement Facts** | | | |
|---|---|---|---|
| **Serving Size 250ml** | | | |
| | Amount Per Serving | | % DV ** |
| **Calories** | 379 | kcal | |
| | | | |
| **Total Fat** | 19 | g | 24% |
| **Cholesterol** | - | mg | 0% |
| | | | |
| **Total Carbohydrate** | 32 | g | 12% |
| Dietary Fiber | 2 | g | 7% |
| Sugars | 30 | g | |
| **Protein** | 20 | g | 40% |
| **Vitamin A** | 320 | mcg | 35% |
| **Vitamin C** | 480 | mg | 35% |
| **Vitamin D** | 7 | mcg | 35% |
| **Vitamin E** | 5 | mg | 35% |
| **Vitamin K** | 42 | mcg | 35% |
| **Thiamine** | 400 | mcg | 35% |
| **Riboflavin** | 450 | mcg | 35% |
| **Niacin** | 6 | mg | 35% |
| **Vitamin B6** | 600 | mcg | 35% |
| **Folate** | 132 | mcg | 35% |
| **Vitamin B12** | 1 | mcg | 35% |
| **Biotin** | 10 | mcg | 35% |
| **Pantothenic Acid** | 2 | mg | 35% |
| **Calcium** | 480 | mg | 35% |
| **Iron** | 6 | mg | 35% |
| **Phosphorus** | 450 | mg | 35% |
| **Chloride** | - | mg | † |
| **Sodium** | 175 | mg | 7% |
| **Potassium** | 1,600 | mg | 35% |

Sensory Evaluation. FFAs produced from the vegetable oil blend were found to be unpalatable by a taste panel. Surprisingly, the MAG oil produced from this blend was palatable and similar in the taste and texture of the original triglyceride oil. When the MAG oil was formulated into the RTD product described above, the flavor was acceptable and indistinguishable from similar commercial products with intact (un-digested) lipids and proteins.

### Example 5: Production of multiple batches of EMO

The timing of the process described in Example 1 for manufacturing EMO was evaluated to generate oil that was greater than 70% MAG, greater than >85% MAG + FFA and TAG content of 5% or less.

**Table 2 depicts a scan of TLC plates illustrating the concentrations of MAG and FFA in the enzyme modified EMO produced from an olive oil/flax oil/sunflower oil blend (ratio of 7/2/1). In these experiments, step 2 was extended to 84 hours to establish upper limits on timing and temperature to avoid TAG formation.**

| Experiment | TAG | DAG | MAG | FFA | FFA + MAG |
|---|---|---|---|---|---|
| 1 | 4 | 26 | 56 | 13 | 70 |
| 2 | 4 | 11 | 73 | 13 | 85 |
| 3 | 5 | 10 | 76 | 9 | 85 |

The reaction can be monitored in essentially real time with TLC analysis and stopped at any point during step 2 to yield to desired amounts of MAG, DAG, TAG and FFA. Reaction time of 72 hours for step 2 was found to be a practical and productive stopping point and illustrated in Example 6.

### Example 6: Evaluation of TAG-Free Enzyme Modified Almond Oil by NMR Analysis.

TAG-free EMO was produced using almond oil. Reaction conditions were as described in Example 1. Step 2 was 72 hours to minimize TAG production.

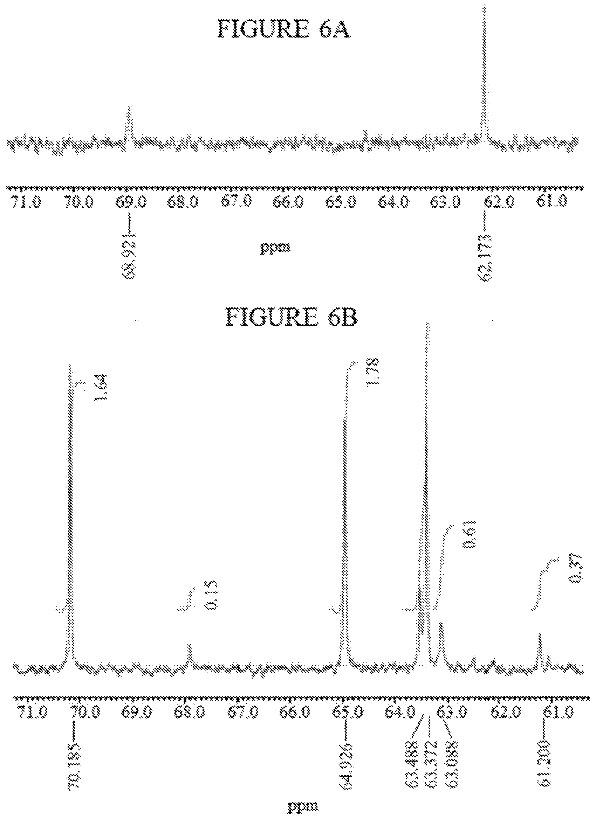

FIGURES 6A-6B depict the ¹³C-NMR analysis of the EMO produced from almond oil. FIGURE 6A illustrates that ¹³C-NMR signal associated with authentic TAG (tristerin) and the characteristic peaks at 62.173 ppm and 68.921 ppm. FIGURE 6B illustrates the ¹³C-NMR signal for the EMO. It shows that there is no discernable TAG signals. Integration of the actual signals indicates an acyl glycerol distribution between MAG:DAG of 88%:12%. Samples were analyzed on a JEOL model ECA600II NMR spectrometer operating at 600 MHz proton and 150 MHz carbon. Samples were made up in 5 mm tubes and run locked with CDCl3 at ambient temperature.

Therefore, the present invention is well adapted to attain the ends and advantages mentioned as well as those that are inherent therein. While numerous changes may be made by those skilled in the art, such changes are encompassed within the spirit of this invention as illustrated by the appended claims.

## Claims

1. A processed oil derived from an oil source, wherein the processed oil comprises (1) a MAG content equal to or greater than 40% by weight of the total weight of the processed oil; (2) a TAG content equal to or lesser than 5% by weight of the total weight of the processed oil, and (3) non-oil ingredients derived from and naturally present in the oil source, such that said non-oil ingredients are not added to the processed oil, wherein the non-oil ingredients are selected from α-tocopherol, β-tocopherol, δ-tocopherol, γ-tocopherol, α-tocotrienol, β-tocotrienol, δ-tocotrienol, and γ-tocotrienol.

2. The processed oil of claim 1, wherein said oil source is from an origin selected from a plant, an animal, algae or fish.

3. The processed oil of claim 1, wherein said oil source is of plant origin.

4. The processed oil of claim 1, wherein said oil source is selected from the group consisting of olive oil, sunflower oil, corn oil, almond oil, rapeseed oil, palm oil, soybean oil, flaxseed oil, and mixtures thereof.

5. The processed oil of any one of claims 1-4, comprising a MAG content of from about 50% to about 95% by weight based on the total weight of the processed oil.

## Patentansprüche

1. Verarbeitetes Öl, das von einer Ölquelle stammt, wobei das verarbeitete Öl (1) einen MAG-Gehalt gleich oder größer als 40 Gew.-% des Gesamtgewichts des verarbeiteten Öls umfasst; (2) einen TAG-Gehalt, der gleich oder geringer als 5 Gew.-% des Gesamtgewichts des verarbeiteten Öls beträgt, und (3) Nicht-Öl-Bestandteile, die von der Ölquelle abgeleitet sind und natürlich in der Ölquelle vorhanden sind, so dass die Nicht-Öl-Bestandteile dem verarbeiteten Öl nicht zugesetzt werden, wobei die Nicht-Öl-Bestandteile ausgewählt sind aus α-Tocopherol, β-Tocopherol, δ-Tocopherol, γ-Tocopherol, α-Tocotrienol, β-Tocotrienol, δ-Tocotrienol und γ-Tocotrienol.

2. Verarbeitetes Öl nach Anspruch 1, wobei die Ölquelle aus einem Ursprung stammt, der aus einer Pflanze, einem Tier, einer Alge oder einem Fisch ausgewählt ist.

3. Verarbeitetes Öl nach Anspruch 1, wobei die Ölquelle pflanzlichen Ursprungs ist.

4. Verarbeitetes Öl nach Anspruch 1, wobei die Ölquelle ausgewählt ist aus der Gruppe bestehend aus Olivenöl, Sonnenblumenöl, Maisöl, Mandelöl, Rapsöl, Palmöl, Sojabohnenöl, Leinsamenöl und Mischungen davon.

5. Verarbeitetes Öl nach einem der Ansprüche 1-4, das einen MAG-Gehalt von etwa 50 bis etwa 95 Gew.-%, basierend auf dem Gesamtgewicht des verarbeiteten Öls, umfasst.

## Revendications

1. Huile transformée dérivée d'une source oléagineuse, dans laquelle l'huile transformée comprend (1) une teneur en MAG supérieure ou égale à 40 % en poids du poids total de l'huile transformée ; (2) une teneur en TAG inférieure ou égale à 5 % en poids du poids total de l'huile transformée, et (3) des ingrédients non oléagineux dérivés de et naturellement présents dans la source oléagineuse, de sorte que lesdits ingrédients non oléagineux ne sont pas ajoutés à l'huile transformée, dans laquelle les ingrédients non oléagineux sont choisis parmi le α-tocophérol, le β-tocophérol, le δ-tocophérol, le γ-tocophérol, le α-tocotriénol, le β-tocotriénol, le δ-tocotriénol et le γ-tocotriénol.

2. Huile transformée selon la revendication 1, dans laquelle ladite source oléagineuse provient d'une origine choisie parmi une plante, un animal, une algue ou un poisson.

3. Huile transformée selon la revendication 1, dans laquelle ladite source oléagineuse est d'origine végétale.

4. Huile transformée selon la revendication 1, dans laquelle ladite source oléagineuse est choisie dans le groupe consistant en l'huile d'olive, l'huile de tournesol, l'huile de maïs, l'huile d'amande, l'huile de colza, l'huile de palme, l'huile de soja, l'huile de lin et des mélanges de celles-ci.

5. Huile transformée selon l'une quelconque des revendications 1-4, comprenant une teneur en MAG d'environ 50 % à environ 95 % en poids sur la base du poids total de l'huile transformée.
